# EUROPEAN PATENT APPLICATION

(11) **EP 4 643 845 A1**
(43) Date of publication of application: **05.11.2025**
(21) Application number: 23910811.1
(22) Date of filing: 28.12.2023
(51) Int. Cl.: A61K 8/64, A61K 8/36, A61K 8/34, A61Q 19/00, A61Q 17/00

(54) **COSMETIC COMPOSITION**

(30) Priority: 30.12.2022 CN 202211723366
(71) Applicant: Shiseido Company, Ltd., Chuo-ku Tokyo 104-0061 (JP)
(72) Inventor: HAN, Yang, Beijing 100176 (CN); QU, Rui, Beijing 100176 (CN)
(74) Representative: Ter Meer Steinmeister & Partner
(86) International application number: PCT/CN2023/142650
(87) International publication number: WO 2024/140894

(57) **Abstract**

The present invention relates to a cosmetic composition comprising the following components: a lipopeptide compound having a cyclic structure or a salt thereof (A), glycerin (B), a long-chain fatty acid having a branched structure (C), isostearyl alcohol (D), and water, wherein, on the basis of the total mass of the composition, the content of component (A) is greater than 0 and less than 4 mass%.

## Description

### TECHNICAL FIELD

The present disclosure belongs to the field of cosmetics. Specifically, the present disclosure relates to a cosmetic composition that exhibits good transparency in appearance and allows for good permeability of biologically active substances.

### BACKGROUND

Cosmetic composition for external use on the skin can not only confer a favorable appearance (whitening, blemish coverage, artistic makeup or the like), but also serve to inhibit skin imperfections, improve skin nutritional status, and improve or alleviate skin damage caused by superficial trauma and skin-aging.

Therefore, it is often desired to incorporate functional components acceptable to the human body (typically adding bioactive agents) in cosmetic compositions for external use on the skin, so as to achieve the enhancement and preservation of skin health.

Further, as a method for achieving the above purposes, reference or attempts have been made to employ a mechanism similar to a transdermal delivery system, that is, a skin external agent containing a functional component is brought into contact with the skin, such that the functional component is purposefully delivered to a target site. In this case, the functional component is not only required to exhibit good dispersibility but also expected to possess good skin permeability.

Additionally, it is also well-known that cosmetic compositions for external use on the skin are commonly present and used in the form of an aqueous phase system, an oil phase system or a water-oil biphasic system. In particular, in order to maintain necessary stability in the water-oil biphasic system, a surfactant is an essential component, which takes advantage of the affinity of the aqueous or oil phase to allow the dispersed phase particles in the emulsion to exhibit good emulsion stability.

Therefore, as a workable attempt, surfactants have been studied as auxiliary components to improve permeability or absorption of functional components (especially biologically active components) or pharmaceutical ingredients into human skin. For example, in Cited Literature 1, sucrose fatty acid ester as a nonionic surfactant is used as an auxiliary component for promoting transdermal absorption of a drug. Moreover, it has excellent biodegradability, little skin irritation, and an excellent effect of promoting transdermal absorption of molecular drug compounds.

Furthermore, as surfactants with an emulsifying function, pursuit of better emulsion stability, low toxicity, and environmental friendliness has become increasingly evident in cosmetic compositions for external use on the skin. Consequently, bio-derived surfactants have received more and more attention.

Bio-derived surfactants can be broadly categorized into lipopeptides, glycolipids, phospholipids, other neutral lipids, etc. Cited Literature 2 discloses a green bio-surfactant with strong emulsifying functions -- sodium surfactin, which can be obtained by biological fermentation and has been proved to have excellent dispersibility and emulsion stability, and to be less irritating to the human body as compared to other commonly-used surfactants (SDS, etc.).

Furthermore, the ability of lipopeptides containing a cyclic structure to promote transdermal delivery of biologically active substances has also been corroborated in Cited Literature 3. The results show that in the Franz diffusion cell assay, every 1% by weight of the lipopeptide compound or a salt thereof allows the skin permeability of a physiologically active ingredient to be increased by more than twice as compared to the case where the lipopeptide compound or a salt thereof is not added.

In the other aspect, in addition to the pursuit of comfort and functionality in use from cosmetic compositions for external use on the skin, there is also a growing concern with the aesthetic feeling and personalized demands of the appearance provided by the products in modern business. It is generally believed that cosmetics providing people with a refreshing and crystal-clear appearance can cater to consumer preferences in many cases.

While the aforementioned studies have already been conducted in the prior arts, there is still room for improvement in the properties such as functionality and external appearance of cosmetic compositions for external use on the skin.

### Cited Literatures

Cited Literature 1: JP2003-238446A
Cited Literature 2: Zou Qingqing et al., "Green Surfactant with Unique Cyclic Peptides -- KANEKA·Surfactin", New Technologies & Products, February, 2016
Cited Literature 3: JP5906194B2

### SUMMARY

### Technical Problem

As described above, although the usability of cyclic lipopeptides with strong emulsifying properties, environmental friendliness, and low irritation to the human body has already been studied to some extent, the following issues have been found in the production practice of cosmetic compositions:

When the aforementioned lipopeptide compound having a cyclic structure or a salt thereof is used as an emulsifier, for example, as in as in Cited Literature 3, it is generally used in combination with a moisturizing agent contain certain components, such as glycerin (the moisturizing agent may be used as a solvent of the lipopeptide compound by extrapolation). However, when combined in the above manner, the desired effect of adequately promoting the skin permeability of biologically active ingredients seems not be showed in the multi-component cosmetic composition.

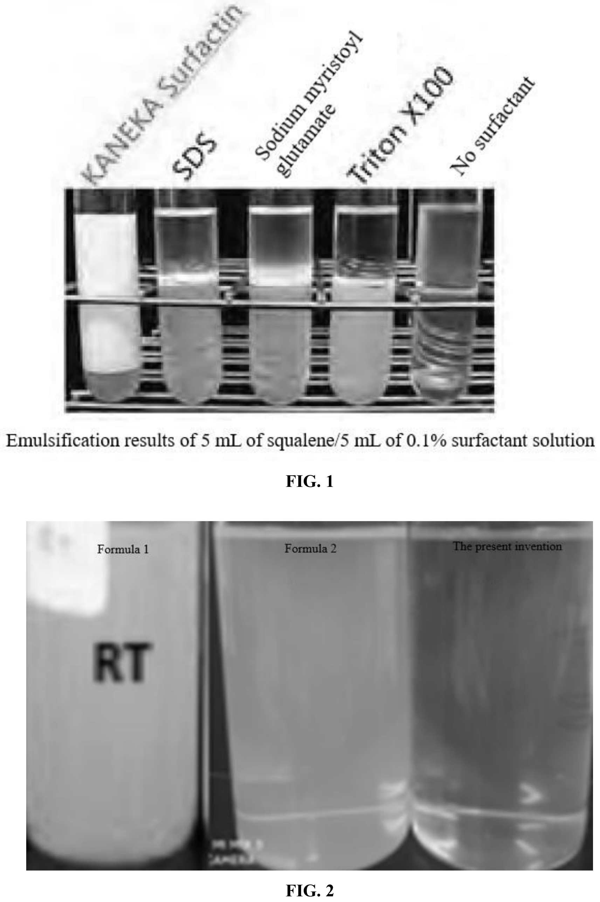

Furthermore, as disclosed in Cited Literature 2, the present disclosure prepares emulsion systems containing various surfactants as shown in FIG. 1 (*i.e.* FIG. 2 of Cited Literature 2). The emulsion system, in which the lipopeptide compound having a cyclic structure or a salt thereof (KANEKA·surfactin) is used, exhibits high emulsifying properties and emulsion stability. Nonetheless, it is because of its high emulsifying properties that a distinctly white, opaque appearance occurs while forming the emulsion system, which in some cases is detrimental to conferring a refreshing and crystal-clear appearance in the preparation of cosmetic products for external use on the skin.

Accordingly, in view of the above-mentioned problems encountered in this field when preparing cosmetic compositions for external use on the skin by a lipopeptide compound having a cyclic structure or a salt thereof, the present disclosure mainly provides a cosmetic composition containing a lipopeptide compound having a cyclic structure or a salt thereof, which under the coordination of specific components with the lipopeptide compound, can exhibit high transparency even when an additional oil component is further added to the composition to form a distinct emulsion (in particular an oil-in-water emulsion), thereby endowing the product with a refreshing and crystal-clear external appearance, while improving ability of the lipopeptide compound to promote the skin permeability of the biologically active component.

### Solution to Problem

After intensive studies, the present inventors have found that the above-mentioned technical problems can be solved by implementing the following technical solutions:
[1]. The present disclosure firstly provides a cosmetic composition, wherein the composition comprises the following components:
   a lipopeptide compound having a cyclic structure or a salt thereof (A);
   glycerin (B);
   a long-chain fatty acid having a branched structure (C);
   isostearyl alcohol (D); and water,
   wherein on the basis of a total mass of the composition:
   the content of the component (A) is more than 0 mass% and 4 mass% or less.
[2]. The composition according to [1], wherein the lipopeptide compound having the cyclic structure or the salt thereof (A) comprises a metal salt of surfactin.
[3]. The composition according to [1] or [2], wherein the long-chain fatty acid having the branched structure (C) is one or more selected from fatty acids having a branched structure and having 14 to 24 carbon atoms.
[4]. The composition according to any one of [1] to [3], wherein
   the content of the component (B) is more than 0 mass% and 30 mass% or less;
   the content of the component (C) is more than 0 mass% and 2 mass% or less; and
   the content of the component (D) is more than 0 mass% and 2 mass% or less.
[5]. The composition according to any one of [1] to [4], wherein the composition further comprises a diol compound (E).
[6]. The composition according to any one of [1] to [5], wherein the composition further comprises a biologically active component and/or an oil component.
[7]. A preparation method for the composition according to any one of [1] to [6], wherein the method comprises mixing a first raw material portion with a second raw material portion, wherein
   the first raw material portion contains water,
   the second raw material portion contains the components (A), (C), and (D), and
   the component (B) is distributed in the first raw material portion and/or the second raw material portion in any proportion.
[8]. The preparation method according to [7], wherein in the second raw material portion, the mass of the component (C) is 0.20 to 0.80 times the mass of the component (A) and/or the amount of the component (D) may be 0.20 to 0.80 times the amount of the component (A).
[9]. The composition according to any one of [1] to [6] or a composition obtained by the preparation method according to [7] or [8], which has an L value of 90 or more.
[10]. The composition according to any one of [1] to [6] or a composition obtained by the preparation method according to [7] or [8], wherein the composition is an emulsion system.

### Advantageous Effects of the Invention

By implementing the above-mentioned technical solutions, the present disclosure could achieve the following technical effects:
1) The cosmetic compositions provided by the present disclosure are excellent in transparency, capable of providing users with a refreshing and non-greasy feeling and use experience, and capable of retaining a high level of transparency even when an oil component is additionally added (to form a distinct emulsion).
2) By using specific components, such as a long-chain fatty acid having a branched structure (C), an isostearyl alcohol (D) and glycerin in combination with a lipopeptide compound having a cyclic structure or a salt thereof (A), the cosmetic compositions provided by the present disclosure not only retain good surface activity and antibacterial properties but also can still achieve excellent transparency, while having the ability to achieve a further improved effect of promoting the transdermal absorption of functional components, particularly biologically active components as compared to the prior art.

### BRIEF DESCRIPTION OF THE DRAWINGS

FIG. 1: studies on emulsifying properties of different surfactants in the prior arts
FIG. 2: comparison of appearances between the prior-art formulas and the composition of the present disclosure
FIG. 3: simulation of the technical solution in an example of the prior art

### DETAILED DESCRIPTION

The present disclosure will be described in detail below. The technical features described below are explained based on the representative embodiments and specific examples of the present disclosure, but the present disclosure is not limited thereto. It should be noted that:

The numerical range represented by "numerical value A to numerical value B" used in the present specification refers to the range including the endpoint values A and B.

In the present specification, the term "basically" or "substantially" means that the standard deviation from the theoretical model or theoretical data is within a range of 3%, preferably 2%, more preferably 1%. Moreover, the deviation here also includes a systematic deviation.

In the present specification, the term "may" involves both the meaning of doing something and the meaning of not doing something.

In the present specification, the term "normal temperature" or "room temperature" refers to an indoor temperature at 23 ± 2°C.

In the present specification, the terms "first raw material portion" and "second raw material portion" are named only for distinguishing them from each other, in which "first" and "second" do not involve a meaning or concept concerning the order.

In the present specification, the term "optional" or "optionally" means that the event or case described below may or may not occur, and this description includes the case where this event occurs and the case where this event does not occur.

Phrases such as "some specific/preferred embodiments", "other specific/preferred embodiments", "embodiments" and the like referred to in the present specification mean that particular elements (for example, features, structures, properties and/or characteristics) described in relation to this embodiment are included in at least one of the embodiments described herein, and may or may not exist in other embodiments. Additionally, it should be appreciated that the elements may be combined in any suitable manner into various embodiments.

The present disclosure provides, first and foremost, a cosmetic composition for external use on the skin which is prepared from a lipopeptide compound having a cyclic structure or a salt thereof. With the aid of specific components (a long-chain fatty acid having a branched structure (C) and an isostearyl alcohol (D)) together with glycerin in the composition, not only does the lipopeptide compound having a cyclic structure or a salt thereof (A) retain its good surface activity and antibacterial properties, but also the resulting composition can still achieve excellent transparency, while having the ability to achieve a further improved effect of promoting the transdermal absorption of functional components, particularly biologically active components as compared to the prior art.

### <First Aspect>

In the first aspect of the present disclosure, there is provided a cosmetic composition for external use on the skin. The composition comprises the following components as essential components: a lipopeptide compound having a cyclic structure or a salt thereof (A); glycerin (B); a long-chain fatty acid having a branched structure (C); an isostearyl alcohol (D); and water.

In the present disclosure, the addition of the above-mentioned components (B) to (D) can not only improve the effect of the component (A) on the human skin permeability of a biologically active substance that is potentially used, but also retain the transparency of the composition even when an additional oil component is used.

### Component (A)

The component (A) of the present disclosure is a biosurfactant having a surface activity, and specifically a lipopeptide-based surfactant having a cyclic structure.

The specific structure of the aforementioned lipopeptide-based surfactant is not particularly limited in the present disclosure. For the specific structures of these substances or salts thereof, the present disclosure cites the substance represented by formula (1) disclosed in JP5906194B2, which constitutes prior art.

Furthermore, the salts of the aforementioned lipopeptide compound having a cyclic structure may be various metal salts, and in the present disclosure, they are preferably one or more of alkali metal salts, alkaline earth metal salts, or ammonium salts, more preferably alkali metal salts, in particular sodium salts.

The component (A) of the present disclosure can be, in principle, obtained by microbial fermentation techniques. In some preferred embodiments of the present disclosure, it may be produced from the microorganism *Bacillus Subtilis* by fermentation techniques, thereby obtaining an anionic lipopeptide surfactant having a cyclic structure, which is extremely water soluble and can be represented by the following structural formula (1), but the length of the alkyl chain and the position of the branched chain are not limited to those in structural formula (1), and main comprise a structure in which one amino acid is substituted with another amino acid:

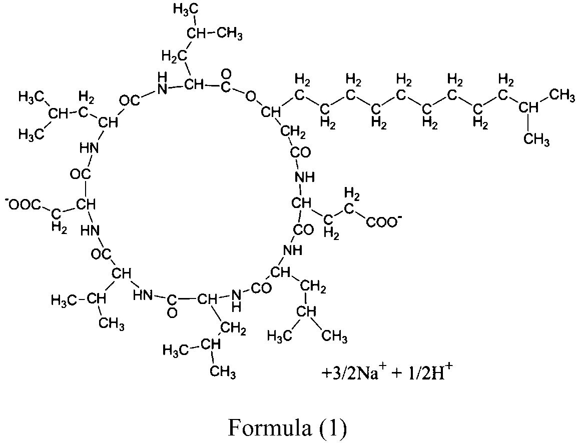

The structure of formula (1) is formed by attaching a cyclic peptide hydrophilic group consisting of seven amino acids to a β-hydroxy fatty acid. Due to the unique cyclic peptide structure, when micelles are formed in water, hydrogen bonds can be formed between molecules, which allow it to exhibit a stronger binding ability as compared to other surfactants because it can form a polymer with an exceptionally low number of molecules. It is for this reason that according to relevant reports, the critical micelle concentration (CMC) of the above structure is only 0.0003% (mass fraction), and this structure is one of the surfactants having the lowest CMC and shows strong emulsifying properties.

As for the aforementioned lipopeptide compound having a cyclic structure or a salt thereof, a mixture of one or more thereof may be obtained by biological fermentation as described above or may be commercially available (KANEKA·surfactin).

Furthermore, as to the amount of the component (A) in the cosmetic composition of the present disclosure, in some specific embodiments, the amount of the component (A) may be 4 mass% or less, preferably 3.5 mass% or less, further preferably 3 mass% or less on the basis of the total mass of the cosmetic composition. If the amount of the component (A) is excessive, it not only leads to an undesirable increase in production cost, but also may lead to an unfavorable feeling to the skin, causing an astringent feeling after the cosmetic is applied to the skin. In addition, excessive use of the component (A) may also result in excessive foam due to its strong emulsifying properties and thus affect the fillability of the cosmetic and reduce the transparency in appearance.

The lower limit of the amount of the component (A) is not particularly limited and may generally be more than 0 mass%, preferably 0.0001 mass% or more, further preferably 0.001 mass% or more, more preferably 0.01 mass% or more, still more preferably 0.1 mass% or more, 0.2 mass% or more, etc.

### Component (B)

The component (B) of the present disclosure is a glycerin component. In general cases, the glycerin component is used as a moisturizing agent or a solvent. In the present disclosure, glycerin can be used to dissolve the lipopeptide compound having a cyclic structure or a salt thereof (A) or used as a carrier thereof.

Therefore, the component (B) may be added to the composition alone or mixed with the other components in the composition. Alternatively, the component (B) may be mixed with the component (A) and then mixed with the other components to yield the cosmetic composition of the present disclosure.

As to the amount of the component (B) in the present disclosure, in some specific embodiments, the amount of the component (B) may be 30 mass% or less, preferably 25 mass% or less, further preferably 20 mass% or less or 18 mass% or less on the basis of the total mass of the cosmetic composition. If the amount of the component (B) is excessive, it may also lead to an unfavorable feeling to skin, causing stickiness after the cosmetic is applied to the skin.

The lower limit of the amount of the component (B) is not particularly limited, and may generally be more than 0 mass%, preferably 0.0003 mass% or more, further preferably 0.003 mass% or more, more preferably 0.01 mass% or more, still more preferably 0.10 mass% or more, 0.20 mass% or more, 1.00 mass% or more, 3.00 mass% or more, etc. If the amount of the component (B) is too small, it may result in poor permeability and weakened transparency of the system.

Additionally, in some preferred embodiments of the present disclosure, at least part of the component (B) may be used together as a solvent or carrier of the component (A) (*i.e.* as a raw material, they are mixed and then added to the composition simultaneously). In this case, in the mixture formed of component (A) and component (B), the amount of the component (B) is 1.0 to 4.0 times, preferably 1.5 to 3.5 times the mass of the component (A). It is believed in the present disclosure that such a compounding ratio can increase the dispersibility of the component (A) in the cosmetic composition, which is advantageous for the component (A) to exert its effect of promoting skin permeability of the biologically active component that is potentially used.

Furthermore, in other specific embodiments of the present disclosure, the component (B) may be used only as a solvent or carrier of the component (A), or may be used partially as a solvent or carrier of the component (A) while the other portion of component (B) is directly or separately added to the cosmetic composition.

Furthermore, the present disclosure has found that incorporation of the following components (C) and (D) into the cosmetic composition containing the above-mentioned components (A) and (B) can not only improve the effect of the component (A) on the skin permeability of the biologically active component that is potentially used, but also unexpectedly impart the resulting composition with a previously unattainable, appealing transparency in appearance.

Components (C) and (D) will be described below, respectively.

### Component (C)

The component (C) of the present disclosure may be a long-chain fatty acid having a branched structure, preferably a saturated fatty acid.

The "long chain" according to the present disclosure refers to an aliphatic chain having more than 12 carbon atoms. In some specific embodiments of the present disclosure, the component (C) of the present disclosure may be a fatty acid having a branched structure and having 14 to 24 carbon atoms. In the preferred embodiments of the present disclosure, the component (C) may be isostearic acid or isopalmitic acid, more preferably isostearic acid.

As to the amount of the component (C) in the present disclosure, in some specific embodiments, the amount of the component (C) may be 2 mass% or less, preferably 1.8 mass% or less, 1.5 mass% or less, further preferably 1.0 mass% or less on the basis of the total mass of the cosmetic composition. If the amount of the component (C) is excessive, the effect of the cosmetic composition to promote the permeability may not be significantly improved and the transparency of the cosmetic composition may also be impaired.

The lower limit of the amount of the component (C) is not particularly limited, and may generally be more than 0 mass%, preferably 0.00002 mass% or more, further preferably 0.003 mass% or more, more preferably 0.01 mass% or more, still more preferably 0.02 mass% or more, 0.05 mass% or more, 0.10 mass% or more, etc.

Additionally, from the standpoint of improving the effect of component (A) to promote skin permeability of biologically active substances, in some preferred embodiments of the present disclosure, the amount of the component (C) may be 0.20 to 0.80 times, further preferably 0.25 to 0.60 times the amount of component (A).

### Component (D)

The component (D) of the present disclosure may be isostearyl alcohol. As to the amount of the component (D) in the present disclosure, in some specific embodiments, the amount of the component (D) may be 2 mass% or less, preferably 1.8 mass% or less, 1.5 mass% or less, further preferably 1.0 mass% or less on the basis of the total mass of the cosmetic composition. If the amount of the component (D) is excessive, the effect of the cosmetic composition to promote the permeability may not be significantly improved and the transparency of the cosmetic composition may also be impaired.

The lower limit of the amount of the component (D) is not particularly limited, and may generally be more than 0 mass%, preferably 0.00002 mass% or more, further preferably 0.003 mass% or more, more preferably 0.01 mass% or more, still more preferably 0.02 mass% or more, 0.05 mass% or more, 0.10 mass% or more, etc.

Additionally, from the standpoint of improving the effect of component (A) to promote skin permeability of biologically active substances, in some preferred embodiments of the present disclosure, the amount of the component (D) may be 0.20 to 0.80 times, further preferably 0.25 to 0.60 times the amount of the component (A).

In addition to the above-mentioned essential components, the cosmetic composition according to the present disclosure may further contain one or more of the following components as necessary:

### Component (E)

The component (E) of the present application is a diol compound. The diol compounds usable in the present disclosure are not particularly limited in principle, and may be various diols commonly used in the field of cosmetics.

Component (E) may partially function as a moisturizing agent/solvent. Therefore, one the one hand, the component (E) may be added to the composition alone or mixed with the other components of the composition, or at least partially mixed with the component (A) and then mixed with the other components to yield the cosmetic composition of the present disclosure; on the other hand, where a certain amount of component (E) is used, the amount of component (B) may be reduced.

In some specific embodiments, the component (E) of the present disclosure may be an alkylene glycol having a branched chain or a branched structure and having 2 to 8, preferably 3 to 6 carbon atoms. Specific examples may include: a mixture of one or more diols selected from propanediol, butanediol, isopentyldiol, 1,2-hexanediol, etc.

The amount of the component (E) in the present disclosure is not particularly limited in principle. In some specific embodiments, the amount of the component (E) may be 25 mass% or less, preferably 20 mass% or less, still preferably 18 mass% or less, further preferably 15 mass% or less, more preferably 10 mass% or less on the basis of the total mass of the cosmetic composition. If the amount of the component (E) is excessive, this may lead to poor skin permeability of biologically active substances potentially present in the cosmetic composition or impairment of persistence of the transparency of the resulting cosmetic composition.

The lower limit of the amount of the component (E) is not particularly limited, and may generally be more than 0 mass%, preferably 0.00002 mass% or more, further preferably 0.003 mass% or more, more preferably 0.01 mass% or more, still more preferably 0.10 mass% or more, 0.50 mass% or more, 1.00 mass% or more, 2.00 mass% or more, etc.

### Biologically Active Substances

As described above, in terms of a function of the cosmetic composition of the present disclosure, if containing a biologically active substance, the composition having specific components according to the present disclosure could improve the effect of the lipopeptide compound on the human skin permeability of these biologically active substances.

The biologically active substances usable in the present disclosure are not particularly limited in principle as long as their incorporation does not damage the transparency of the composition of the present disclosure.

Examples of the usable biologically active substances include one or more of an antioxidant ingredient, a (biological) moisturizing ingredient, an anti-aging ingredient, a cell repair ingredient, a cell nutrient ingredient, a whitening-promoting ingredient, an antibacterial ingredient, an anti-allergic ingredient, a vitamin ingredient, etc.

Furthermore, specific examples of these biologically active substances include: arbutin and derivatives thereof, L-ascorbic acid and derivatives thereof (such as ascorbyl phosphate), hydroquinone and derivatives thereof, glutathione and derivatives thereof, pantothenic acid and derivatives thereof, tranexamic acid and derivatives thereof, kojic acid and derivatives thereof, cysteine and derivatives thereof, ellagic acid and derivatives thereof, resorcinol derivatives, plant extracts such as chamomile extract and licorice extract, superoxide dismutase and derivatives thereof, mannitol and derivatives thereof, rutin and derivatives thereof, bilirubin and derivatives thereof, tryptophan and derivatives thereof, histidine and derivatives thereof, flavones such as quercetin and quercitrin, catechin and derivatives thereof, gallic acid and derivatives thereof, vitamin C and derivatives thereof (isoascorbic acid and derivatives thereof), sesamin and derivatives thereof, α-lipoic acid and derivatives thereof, glycyrrhizic acid and derivatives thereof (glycyrrhizic acid, such as dipotassium glycyrrhizinate and ammonium glycyrrhizinate), thiotaurine and derivatives thereof, urea and derivatives thereof, glycerin and derivatives thereof, xylitol and derivatives thereof, erythritol and derivatives thereof, salicylic acid and derivatives thereof (such as salicylate and 4-methoxysalicylic acid), nicotine and derivatives thereof, nicotinic acid and derivatives thereof (such as nicotinate and nicotinamide), hydroxyproline and derivatives thereof, serine and derivatives thereof, glutamine and derivatives thereof (such as glutamic acid, pyroglutamic acid, pyroglutamic acid, and L-theanine), trimethylglycine and derivatives thereof, arginine and derivatives thereof, alanine and derivatives thereof, adenosine and derivatives thereof, lysine and derivatives thereof, lignans and derivatives thereof, minoxidil and derivatives thereof, finasteride and derivatives thereof, flavanones, tannic acid and derivatives thereof, ferulic acid and derivatives thereof, fullerene and derivatives thereof, 1-(hydroxymethyl)-5,5-dimethylhydantoin and derivatives thereof, D-amino acid and derivatives thereof, oligosaccharide and derivatives thereof, D-glucosamine and derivatives thereof (such as chitin and chitosan), sodium chondroitin sulfate and derivatives thereof, sorbitol and derivatives thereof, trehalose and derivatives thereof, hyaluronic acid and derivatives thereof, maltitol and derivatives thereof, raffinose and derivatives thereof, alginic acid and derivatives thereof, caffeine and derivatives thereof, keratin hydrolysate and derivatives thereof, silk protein hydrolysate and derivatives thereof, azelaic acid and derivatives thereof, γ-aminobutyric acid and derivatives thereof, allantoin and derivatives thereof, γ-oryzanol and derivatives thereof, L-carnitine and derivatives thereof, β-1,3-glucan and derivatives thereof, biotin and derivatives thereof, pyridoxine hydrochloride and derivatives thereof, royal jelly such as propolis, etc.

The amount of the above-mentioned biologically active substance is not particularly limited in principle as long as its amount does not impair the transparency of the cosmetic composition. In some specific embodiments of the present disclosure, the amount of the biologically active substance may be 0.01 mass% to 1000 mass%, preferably 0.10 mass% to 500 mass%, more preferably 0.20 mass% to 300 mass% of the amount of the component (A).

### Oil Component

In the cosmetic composition of the present disclosure, a certain amount of oil component may be further added, so that the composition is present in the emulsion form. As described above, under the cooperation of the specific components of the present disclosure, the composition can exhibit a transparent appearance even when it is present in the emulsion form.

The oil component usable in the composition of the present disclosure is not particularly limited and may be selected from the scope of substances with the transparency commonly used in the cosmetics. Examples of the preferred oil component may include a mixture of one or more of hydrocarbon oils, silicone oils, ester oils, other liquid greases, etc.

As hydrocarbon oils, oils of alkane or (poly)olefin may be used, and their examples include liquid paraffin, squalane, squalene, paraffin, isoparaffin, ozokerite, vaseline, hydrogenated polydecene, etc.

Examples of silicone oils may be include, for example, chain silicones such as dimethylpolysiloxane, methylphenyl polysiloxane, and methyl hydrogen polysiloxane; cyclic silicones such as octamethylcyclotetrasiloxane, decamethylcyclopentasiloxane, and dodecamethylcyclohexasiloxane; silicone resins and silicone rubbers having a three-dimensional network structure, etc.

The liquid greases may include those greases having a melting point of lower than 50°C, particularly at room temperature or lower, such as mono- or poly-fatty acid esters of glycerin, glycerin polycondensates, and phytosterol esters. Examples of the liquid greases in the present disclosure may include palm oil, palm kernel oil, linseed oil, camellia seed oil, macadamia oil, corn oil, mink oil, olive oil, avocado oil, camellia oil, castor oil, safflower oil, jojoba oil, sunflower oil, almond oil, rapeseed oil, sesame oil, soybean oil, peanut oil, triglycerol, tricaprylin, triisopalmitin, glyceryl monostearate, glyceryl monoisostearate, glyceryl molooleate, glyceryl monohydroxystearate, glyceryl monomyristate, etc.

Examples of the ester oils may include: cetyl octanoate, hexyl laurate, isopropyl myristate, octyl palmitate, isocetyl stearate, isopropyl isostearate, octyl isopalmitate, isodecyl oleate, cetyl ethylhexanoate, glyceryl tri-2-ethylhexanoate, pentaerythritol tetra-2-ethylhexanoate, 2-ethylhexyl succinate, diethyl sebacate, etc.

As for the aforementioned oil components, from the standpoint of maintaining the ultimate transparency of the cosmetic, the content of the oil components may be no more than 2.5 mass%, preferably no more than 2.2 mass%, further preferably no more than 2.0 mass%, more preferably 0.00005 mass% to 1.8 mass%, e.g., 0.001 mass%, 0.01 mass%, 0.1 mass%, 0.2 mass%, 0.5 mass% or 1.0 mass% on the basis of the total mass of the cosmetic composition of the present disclosure.

### Additional Components

In addition to the above-mentioned components, the cosmetic composition of the present disclosure may also comprise other optional components provided that the technical effects of the present disclosure are not adversely affected.

These optional components include the other components than the above-mentioned ones, such as surfactants, moisturizing agents, powders, alcoholic solvents, water-soluble polymers, oil-soluble polymers, film-forming agents, ultraviolet absorbers, preservatives, antibacterial agents, fragrances, salts, antioxidants, pH adjusters, chelating agents, fresheners, anti-inflammatory agents, and skin-beautifying ingredients.

The composition of the present disclosure formed of the aforementioned components can exhibit excellent transparency even in the case of containing an oil component. In some specific embodiments, the cosmetic composition of the present disclosure may have an L value of 90 or more, preferably 95 or more, more preferably 99 or more. Moreover, after storage at 50°C for 3 days, the L value can also be more than 80, preferably 85 or more. This shows the excellent transparency and stability of transparency of the present disclosure. This is because, in general, even the (instant) L value of microemulsions is typically only around 80 and will not exceed 90 in the prior art.

### <Second Aspect>

In the second aspect of the present disclosure, there is provided a preparation method for a cosmetic composition.

The preparation method of the present disclosure may comprise at least a step of mixing a first raw material portion with a second raw material portion.

The first raw material portion comprises at least water. Moreover, in some specific embodiments, the first raw material portion may also include at least part of component (B) and/or at least part of component (E).

The second raw material portion comprises at least component (A), component (C), component (D), at least part or all of component (E). Furthermore, the component (B) may be at least partially present in the second raw material portion, or may be distributed between the first raw material portion and the second raw material portion in any ratio.

In addition, the second raw material portion may also contain at least part of water.

In some preferred embodiments of the present disclosure, in the presence of a biologically active component, the biologically active component may be present in the first raw material portion or the second raw material portion or in both the first and second raw material portions. From the standpoints of ease of operation and compatibility, the biologically active component may be preferably present in the first raw material portion.

In some preferred embodiments of the present disclosure, in the presence of an oil component, the oil component may be present in the first raw material portion or the second raw material portion or in both the first and second raw material portions. Alternatively, from the standpoint of forming a strongly stable emulsion system, at least part of the oil component may be mixed with the mixture of the first raw material portion and the second raw material portion.

Therefore, in further preferred embodiments of the present disclosure:
i) the first raw material portion comprises: water, part of component (B), and part of component (E); and
ii) the second raw material portion comprises: components (A), (C), and (D), the other part of component (B), and the other part of component (E), wherein in the second raw material portion, the amount of the component (B) is 1.0 to 4.0 times, preferably 1.5 to 3.5 times the mass of the component (A); and the content of the component (E) is, on the basis of the total mass of the composition, 2.2 mass% or less, e.g., 2.0 mass% or less or 1.8 mass% or less, and 0.00001 mass% or more, 0.001 mass% or more, 0.01 mass% or more, or 0.1 mass% or more, etc.

In other preferred embodiments of the present disclosure:
i) the first raw material portion comprises: water and part of component (B); and
ii) the second raw material portion comprises: components (A), (C), and (D), part of component (B), and component (E), wherein in the second raw material portion, the amount of the component (B) is 1.0 to 4.0 times, preferably 1.5 to 3.5 times the mass of the component (A); and the content of the component (E) is, on the basis of the total mass of the composition, 2.2 mass% or less, e.g., 2.0 mass% or less or 1.8 mass% or less, and 0.00001 mass% or more, 0.001 mass% or more, 0.01 mass% or more, or 0.1 mass% or more, etc.

Without being restricted, the other raw material portions than the aforementioned raw material portions may further be added optionally when preparing the composition of the present disclosure. For example, at least part of the aforementioned oil component may be used as a separate raw material portion. In addition, the other raw material portions that can be used independently may include those optional additional components of the present disclosure.

With the above-mentioned preparation method, in some specific embodiments, the cosmetic composition of the present disclosure may be an emulsified system (including a microemulsion system), in particular an oil-in-water emulsified system. Preferably, the emulsified system contains emulsion droplets having an average particle size of 10 to 100 nm, and examples of the average particle size may further be 20, 30, 40, 50, 60, 70, 80, 90 nm, etc.

In consideration of production and usage, the cosmetic composition obtained in the present disclosure has a viscosity of 20000 mPa·s or 15000 mPa·s or 10000 mPa·s or 8000 mPa·s or 6000 mPa·s or 5000 mPa·s or 4000 mPa·s or 3000 mPa·s (at room temperature) or less and 1 mPa·s or 10 mPa·s or 20 mPa·s or 30 mPa·s (at room temperature) or more to maintain a good feeling of use without causing stickiness and provide consumers with a refreshing skin feeling. Further preferably, the cosmetic composition has a viscosity of 4000 to 9000 mPa·s (at room temperature).

### Examples

The present disclosure will be further described below with reference to specific examples.

### Test method for the L value:

Samples were filled in a quartz glass dish (optical path length: 10 mm) without dilution and the transmittance was determined at 20°C by an integrating sphere spectrophotometer (x·rite, Inc.).

That is, the samples were uniformly irradiated with light from various angles using an integrating sphere to obtain the ratio (%) of the transmitted light intensity received in the normal direction of the sample surface to the irradiated light intensity, which was defined as the L value.

### Test method for permeability (franz diffusion cell assay):

Test method for carnosine: A permeable membrane (Start M, Merck) was mounted on a Franz cell. The above permeable membrane was filled with pbs buffer at the receiving side and filled with 0.78 ml of samples at the donating side. The permeable conditions were as follows: the diameter was 1 cm and the temperature was at 39°C. After permeation for 24 hours, the content of carnosine in the pbs buffer at the receiving side was determined by HPLC.

Test method for sodium hyaluronate: A permeable membrane (Start M, Merck) was mounted on a Franz cell. The above permeable membrane was filled with pbs buffer at the receiving side and filled with 7.8 µL of samples at the donating side. The permeable conditions were as follows: the diameter was 1 cm and the temperature was at 39°C. After permeation for 24 hours, the content of the sodium hyaluronate in the permeable membrane was determined according to the method prescribed on the ELISA Kit (hyaluronan qkit, DHYALO).

The cosmetic compositions in the following examples, comparative examples, and reference examples were all obtained by the following mixing method:

The components of the second raw material portion were mixed according to the tables, then the second raw material portion was mixed with the first raw material portion, and finally the resulting mixture was mixed with the third raw material portion to ultimately obtain a cosmetic composition. The content of each of the components was in percentage.

### Reference Examples:

The cosmetic compositions in Reference Examples 1 to 3 were obtained according to the following formulae:

**Table 1:**

| | **Reference Example 1** | **Reference Example 2** | **Reference Example 3** |
|---|---|---|---|
| **First Raw Material Portion** | | | |
| Water | to 100 | to 100 | to 100 |
| Glycerin | 10 | | 20 |
| Sodium hyaluronate | | | |
| Carnosine | | | |

| **Second Raw Material Portion** | | | |
|---|---|---|---|
| Sodium surfactin | 0.0001 | 0.0003 | 3 |
| Glycerin | 0.0003 | 0.0009 | 9 |
| Butanediol | 0.00005 | 0.00015 | 1.5 |
| Squalane | | | 1.8 |
| α-Olefin polymer | 0.00006 | 0.00018 | |
| Tocopherol | | | |
| Fragrance | | | |
| Isostearyl alcohol | 0.000045 | 0.000135 | 1.35 |
| Isostearic acid | 0.000045 | 0.000135 | 1.35 |
| Water | 0.00005 | 0.00015 | 1.5 |

| **Third Raw Material Portion** | | | |
|---|---|---|---|
| Dipropylene glycol | 9 | 9 | 9 |
| Phenoxyethanol | 0.5 | 0.5 | 0.5 |
| | | | |
| Transparent Emulsification | ○ | ○ | ○ |
| L value at RT | >99 | >99 | >99 |
| L value (after storage at 50°C for 3 d) | >99 | >99 | 98 |

As could be appreciated from the above reference examples, the technical effects of the present disclosure could be achieved even if the components (A) to (E) of the present disclosure were used in small amounts. In addition, the glycerin could also be allowed to be used in a higher amount.

### Examples

The cosmetic compositions in Examples 1 to 5 were obtained according to the following formulae:

**Table 2:**

| | **Example 1** | **Example 2** | **Example 3** | **Example 4** | **Example 5** |
|---|---|---|---|---|---|
| **First Raw Material Portion** | | | | | |
| Water | to 100 | to 100 | to 100 | to 100 | to 100 |
| Glycerin | 10 | 10 | 10 | 10 | 10 |
| Sodium hyaluronate | | | | | |
| Carnosine | | | | | |

| **Second Raw Material Portion** | | | | | |
|---|---|---|---|---|---|
| Sodium surfactin | 0.3 | 0.3 | 0.3 | 0.3 | 0.3 |
| Glycerin | 0.9 | 0.9 | 0.9 | 0.9 | 0.9 |
| Butanediol | 0.15 | | | | |
| Propanediol | | 0.15 | | | |
| Isopentyldiol | | | 0.15 | | |
| 1,2-Hexanediol | | | | 0.15 | |
| Ethanol | | | | | |
| Squalane | 0.18 | 0.18 | 0.18 | 0.18 | 0.18 |
| α-Olefin polymer | | | | | |
| Tocopherol | | | | | |
| Fragrance | | | | | |
| Isostearyl alcohol | 0.135 | 0.135 | 0.135 | 0.135 | 0.135 |
| Isostearic acid | 0.135 | 0.135 | 0.135 | 0.135 | 0.135 |
| Water | 0.15 | 0.15 | 0.15 | 0.15 | 0.15 |

| **Third Raw Material Portion** | | | | | |
|---|---|---|---|---|---|
| Dipropylene glycol | 9 | 9 | 9 | 9 | 9 |
| Phenoxyethanol | 0.5 | 0.5 | 0.5 | 0.5 | 0.5 |
| | | | | | |
| Transparent Emulsification | ○ | ○ | ○ | ○ | Δ |
| L value at RT | >99 | >99 | >99 | >99 | 90 |
| L value (after storage at 50°C for 3 d) | >99 | >99 | >99 | >99 | 88 |

As could be appreciated from the above examples and comparative examples, the addition of diol (*i.e.* component (E)) to the composition of the present disclosure enabled a cosmetic composition having good transparency and permeability.

The cosmetic compositions in Examples 6 to 10 and Comparative Examples 1 to 4 were obtained according to the following formulae:

**Table 3:**

| | **Ex. 6** | **Ex. 7** | **Ex. 8** | **Ex. 9** | **Ex. 10** | **Comp. Ex. 1** | **Comp. Ex. 2** | **Comp. Ex. 3** | **Comp. Ex. 4** |
|---|---|---|---|---|---|---|---|---|---|
| **First Raw Material Portion** | | | | | | | | | |
| Water | to 100 | to 100 | to 100 | to 100 | to 100 | to 100 | to 100 | to 100 | to 100 |
| Glycerin | 10 | 10 | 10 | 10 | 10 | 10 | 10 | 10 | 10 |
| Sodium hyaluronate | | | | | | | | | |
| Carnosine | | | | | | | | | |

| **Second Raw Material Portion** | | | | | | | | | |
|---|---|---|---|---|---|---|---|---|---|
| Sodium surfactin | 0.3 | 0.3 | 0.3 | 0.3 | 0.3 | 0.3 | 0.3 | 0.3 | 0.3 |
| Glycerin | 0.9 | 0.9 | 0.9 | 0.9 | 0.9 | 0.9 | 0.9 | 0.9 | 0.9 |
| Butanediol | 0.15 | 0.15 | 0.15 | 0.15 | 0.15 | 0.15 | 0.15 | 0.15 | 0.15 |
| α-Olefin polymer | 0.18 | 0.18 | 0.18 | 0.18 | 0.18 | 0.18 | 0.18 | 0.18 | 0.18 |
| Isostearyl alcohol | 0.135 | 0.17 | 0.1 | 0.1 | 0.17 | | 0.135 | | 0.27 |
| Isostearic acid | 0.135 | 0.1 | 0.17 | 0.1 | 0.17 | 0.135 | | 0.27 | |
| Water | 0.15 | 0.15 | 0.15 | 0.15 | 0.15 | 0.15 | 0.15 | 0.15 | 0.15 |

| **Third Raw Material Portion** | | | | | | | | | |
|---|---|---|---|---|---|---|---|---|---|
| Dipropylene glycol | 9 | 9 | 9 | 9 | 9 | 9 | 9 | 9 | 9 |
| Phenoxyethanol | 0.5 | 0.5 | 0.5 | 0.5 | 0.5 | 0.5 | 0.5 | 0.5 | 0.5 |
| | | | | | | | | | |
| Transparent Emulsification | ○ | ○ | ○ | ○ | ○ | × | × | × | × |
| L value at RT | >99 | >99 | >99 | >99 | >99 | X white turbidity | X white turbidity | X white turbidity | X white turbidity |
| L value (after storage at 50°C for 3 d) | >99 | >99 | >99 | >99 | >99 | - | - | - | - |

As could be appreciated from the above examples and comparative examples, the addition of isostearic acid (*i.e.* component (C)) and isostearyl alcohol (*i.e.* component (D)) to the composition of the present disclosure enabled a cosmetic composition having good transparency and permeability.

The cosmetic compositions in Example 1 and Examples 11 to 14 were obtained according to the following formulae:

**Table 4:**

| | **Example 1** | **Example 11** | **Example 12** | **Example 13** | **Example 14** |
|---|---|---|---|---|---|
| **First Raw Material Portion** | | | | | |
| Water | to 100 | to 100 | to 100 | to 100 | to 100 |
| Glycerin | 10 | 10 | 10 | 10 | 10 |
| Sodium hyaluronate | | | | | |
| Carnosine | | | | | |

| **Second Raw Material Portion** | | | | | |
|---|---|---|---|---|---|
| Sodium surfactin | 0.3 | 0.3 | 0.3 | 0.3 | 0.3 |
| Glycerin | 0.9 | 0.9 | 0.9 | 0.9 | 0.9 |
| Butanediol | 0.15 | 0.15 | 0.15 | 0.15 | 0.15 |
| Squalane | 0.18 | | | | |
| α-Olefin polymer | | 0.27 | | 0.18 | 0.18 |
| Tocopherol | | | | 0.02 | |
| Fragrance | | | | | 0.02 |
| Isostearyl alcohol | 0.135 | 0.135 | 0.135 | 0.135 | 0.135 |
| Isostearic acid | 0.135 | 0.135 | 0.135 | 0.135 | 0.135 |
| Water | 0.15 | 0.15 | 0.15 | 0.15 | 0.15 |

| **Third Raw Material Portion** | | | | | |
|---|---|---|---|---|---|
| Dipropylene glycol | 9 | 9 | 9 | 9 | 9 |
| Phenoxyethanol | 0.5 | 0.5 | 0.5 | 0.5 | 0.5 |
| | | | | | |
| Transparent Emulsification | ○ | ○ | ○ | ○ | ○ |
| L value at RT | >99 | >99 | >99 | >99 | >99 |
| L value (after storage at 50°C for 3 d) | >99 | >99 | >99 | >99 | >99 |

The above examples showed that the cosmetic compositions of the present disclosure could exhibit good transparency and permeability even when forming an emulsion system (oil-in-water) in the presence of an oil component.

The cosmetic compositions in Examples 15 and 16 and Reference Example 4 were obtained according to the following formulae:

**Table 5:**

| | **Example 15** | **Example 16** | **Reference Example 4** |
|---|---|---|---|
| **First Raw Material Portion** | | | |
| Water | to 100 | to 100 | to 100 |
| Glycerin | 10 | 10 | 10 |
| Sodium hyaluronate | | | |
| Carnosine | | | |

| **Second Raw Material Portion** | | | |
|---|---|---|---|
| Sodium surfactin | 3 | 3 | 3 |
| Glycerin | 9 | 9 | 9 |
| Butanediol | 1.5 | 1.5 | 1.5 |
| Squalane | 1.8 | 1.8 | 2.7 |
| α-Olefin polymer | | | |
| Isostearyl alcohol | 2 | 1.35 | 1.35 |
| Isostearic acid | 1.35 | 2 | 1.35 |
| Water | 1.5 | 1.5 | 1.5 |

| **Third Raw Material Portion** | | | |
|---|---|---|---|
| Dipropylene glycol | 9 | 9 | 9 |
| Phenoxyethanol | 0.5 | 0.5 | 0.5 |
| | | | |
| Transparent Emulsification | Δ | ○ | Δ |
| L value at RT | 93 | >99 | 87 |
| L value (after storage at 50°C for 3 d) | 78 | X white turbidity | 69 |

The above Examples 15 and 16 showed that when the contents of components (C) and (D) were too high, the retention of transparency and permeability might be adversely affected. Further, Reference Example 4 showed that in the case of forming an emulsion, if the content of the oil component was too high, the transparency might be deteriorated.

The cosmetic compositions in Examples 17 and 18 and Comparative Example 5 were obtained according to the following formulae:

**Table 6:**

| | **Example 17** | **Comparative Example 5** | **Example 18** |
|---|---|---|---|
| **First Raw Material Portion** | | | |
| Water | to 100 | to 100 | to 100 |
| Glycerin | 10 | 10 | 10 |
| Sodium hyaluronate | | | |
| Carnosine | | | |

| **Second Raw Material Portion** | | | |
|---|---|---|---|
| Sodium surfactin | 3 | 4.5 | 3 |
| Glycerin | 9 | 13.5 | 13.5 |
| Butanediol | 1.5 | 2.25 | 2.25 |
| Squalane | 1.8 | | 1.8 |
| α-Olefin polymer | | 2.7 | |
| Isostearyl alcohol | 1.35 | 1.5 | 1.35 |
| Isostearic acid | 1.35 | 1.35 | 1.35 |
| Water | 1.5 | 2.25 | 1.5 |

| **Third Raw Material Portion** | | | |
|---|---|---|---|
| Dipropylene glycol | 9 | 9 | 9 |
| Phenoxyethanol | 0.5 | 0.5 | 0.5 |
| | | | |
| Transparent Emulsification | ○ | Δ | ○ |
| L value at RT | >99 | 80 | >99 |
| L value (after storage at 50°C for 3 d) | 82 | X white turbidity | 62 |

As was clear from the comparisons among the examples and comparative example, when the amount of the sodium surfactin was too high, the usability and transparency of the cosmetic composition might be adversely affected.

The cosmetic compositions in Examples 19 and 20 and Comparative Examples 6 and 7 were obtained according to the following formulae:

**Table 7:**

| | **Example 19** | **Example 20** | **Comp. Ex. 6** | **Comp. Ex. 7** |
|---|---|---|---|---|
| **First Raw Material Portion** | | | | |
| Water | to 100 | to 100 | to 100 | to 100 |
| Glycerin | | | | |
| Sodium hyaluronate | 0.1 | | 0.1 | |
| Carnosine | | 3.5 | | 3.5 |

| **Second Raw Material Portion** | | | | |
|---|---|---|---|---|
| Sodium surfactin | 0.3 | 0.3 | 0.3 | 0.3 |
| Glycerin | 0.9 | 0.9 | | |
| Butanediol | 0.15 | 0.15 | | |
| α-Olefin polymer | 0.18 | 0.18 | | |
| Isostearyl alcohol | 0.135 | 0.135 | | |
| Isostearic acid | 0.135 | 0.135 | | |
| Water | 0.15 | 0.15 | | |
| | | | | |
| Permeability | 3.3a | 11b | a | b |

It followed that the permeability of the active ingredient in Example 18 was 3.3 times that in Comparative Example 6, and the permeability of the active ingredient in Example 20 was 11 times that in Comparative Example 7.

### Morphological comparison of cosmetic formulas:

Cosmetics were obtained according to the formulas in the following tables:

**Table 8:**

| Formula 1 | |
|---|---|
| | |
| Water | to 100 |
| Ethanol | 7 |
| Glycerin | 6 |
| Butanediol | 8 |
| 1,3-Propanediol | 8 |
| Agar | 0.5 |
| Polyglyceryl-10 diisostearate | 0.45 |
| Cocoyl glutamate potassium | 0.07 |
| Citric acid | 0.036 |
| Sodium citrate | 0.064 |
| Sodium hexametaphosphate | 0.02 |
| Monocaprylin | 0.06 |

**Table 9:**

| Formula 2 | |
|---|---|
| | |
| Water | to 100 |
| Glycerin | 8 |
| Dipropylene glycol | 9 |
| Butanediol | 6 |
| PEG-6, PEG-32 | 3 |
| PEG/PPG-14/7 Dimethyl ether | 1.1 |
| Cellulose gum | 0.02 |
| PEG-60 Hydrogenated castor oil | 0.16 |
| Polyglyceryl-2 diisostearate | 0.08 |
| Diphenylsiloxy phenyl trimethicone | 0.18 |
| Isostearyl alcohol | 0.06 |
| Isostearic acid | 0.06 |
| Citric acid | 0.02 |
| Sodium citrate | 0.08 |
| Sodium hexametaphosphate | 0.03 |
| Phenoxyethanol | 0.5 |

Furthermore, FIG. 2 showed the appearances of formula 1, recipe 2, and the cosmetic of the present disclosure, suggesting that the present disclosure could obtain a cosmetic in a state of transparent appearance.

### Morphological comparison with the formula of the prior-art cosmetic:

Example 10 in JP2003277220A was simulated in Table 10 below (only some non-essential excipients were removed):

**Table 10:**

| Ingredient a | |
|---|---|
| Isostearic acid | 1 |
| Glyceryl diisostearate | 4 |
| Octyldodecanol | 2 |
| PEG-60 Hydrogenated castor oil | 0.2 |
| Mineral oil | 3 |
| Jojoba oil | 3 |
| Phytosteryl macadamiate | 1 |
| Isopropyl myristate | 5 |
| Glyceryl tri(2-ethylhexanoate) | 2 |
| Hexadecyl 2-ethylhexanoate | 1.2 |

| Ingredient b | |
|---|---|
| Sodium surfactin | 1 |
| Butanediol | 3 |
| Glycerin | 3 |
| Methylparaben | 0.2 |
| Sodium N-lauroylsarcosinate | 0.1 |
| Acrylate/C₁₀₋₃₀ alkyl acrylate crosslinked polymer | 0.1 |
| Water | 70.2 |
| Arginine | 0.2 |

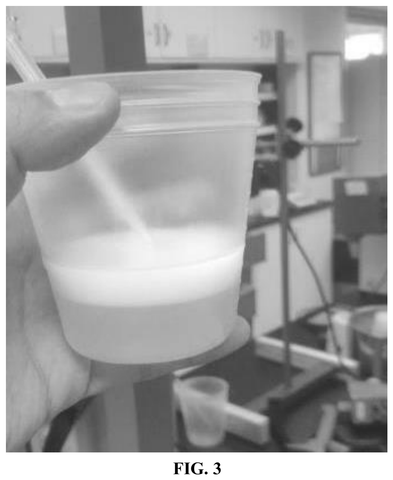

After the cosmetic was obtained by the preparation method in this literature, it was layered within 2 hours, which indicated unstable emulsification. Therefore, this cosmetic could not achieve the transparent appearance of the present disclosure (FIG. 3).

It is to be noted that while the technical solutions of the present disclosure have been introduced with reference to specific examples, a person skilled in the art could understand that the present disclosure is not limited thereto.

Although the examples of the present disclosure have been described above, the above descriptions are exemplary, but not exhaustive; and the disclosed examples are not limiting. A number of variations and modifications may occur to a person skilled in the art without departing from the scopes and spirits of the described examples. The terms in the present disclosure are selected to provide the best explanation on the principles and practical applications of the examples and the technical improvements to the arts on market, or to make the examples described herein understandable to a person skilled in the art.

### Industrial Applicability

The cosmetic compositions provided in the present disclosure can be prepared industrially.

## Claims

1. A cosmetic composition, wherein the composition comprises the following components:
a lipopeptide compound having a cyclic structure or a salt thereof (A);
glycerin (B);
a long-chain fatty acid having a branched structure (C);
isostearyl alcohol (D); and water,
wherein on the basis of a total mass of the composition:
the content of the component (A) is more than 0 mass% and 4 mass% or less.

2. The composition according to claim 1, wherein the lipopeptide compound having the cyclic structure or the salt thereof (A) comprises a metal salt of surfactin.

3. The composition according to claim 1 or 2, wherein the long-chain fatty acid having the branched structure (C) is one or more selected from fatty acids having a branched structure and having 14 to 24 carbon atoms.

4. The composition according to any one of claims 1 to 3, wherein
the content of the component (B) is more than 0 mass% and 30 mass% or less;
the content of the component (C) is more than 0 mass% and 2 mass% or less; and
the content of the component (D) is more than 0 mass% and 2 mass% or less.

5. The composition according to any one of claims 1 to 4, wherein the composition further comprises a diol compound (E).

6. The composition according to any one of claims 1 to 5, wherein the composition further comprises a biologically active component and/or an oil component.

7. A preparation method for the composition according to any one of claims 1 to 6, wherein the method comprises mixing a first raw material portion with a second raw material portion, wherein
the first raw material portion contains water,
the second raw material portion contains the components (A), (C), and (D), and
the component (B) is distributed in the first raw material portion and/or the second raw material portion in any proportion.

8. The preparation method according to claim 7, wherein in the second raw material portion, the mass of the component (C) is 0.20 to 0.80 times the mass of the component (A), and/or
the amount of the component (D) may be 0.20 to 0.80 times the amount of the component (A).

9. The composition according to any one of claims 1 to 6 or a composition obtained by the preparation method according to claim 7 or 8, which has an L value of 90 or more.

10. The composition according to any one of claims 1 to 6 or a composition obtained by the preparation method according to claim 7 or 8, wherein the composition is an emulsion system.
